Europäisches Patentamt

European Patent Office

Office européen des brevets

(11) Publication number: **0 178 443 B1**

(12) **EUROPEAN PATENT SPECIFICATION**

(45) Date of publication of patent specification: **10.06.92**

(51) Int. Cl.⁵: **A61K  7/50**, A61K 7/06, A61K 7/08, A61K 31/715, A61K 35/74

(21) Application number: **85111171.6**

(22) Date of filing: **04.09.85**

(54) Cosmetic and pharmaceutical compositions containing bioemulsifiers.

(30) Priority: **16.10.84 US 662931**

(43) Date of publication of application:
**23.04.86 Bulletin  86/17**

(45) Publication of the grant of the patent:
**10.06.92 Bulletin  92/24**

(84) Designated Contracting States:
**AT BE CH DE FR GB IT LI LU NL SE**

(56) References cited:
**EP-A- 0 016 546**
**EP-A- 0 023 397**

**Chemical Abstracts, vol. 98, 1983, page 415, ref. no. 141447m; Columbus, Ohio, US, E. Rosenberg et al.: "Inhibition of bacterial adherence to hydrocarbons and epithelial cells by emulsan" & Infect. Immun. 1983, 39(3), 1024-8**

(73) Proprietor: **EMULSAN BIOTECHNOLOGIES INC.**
**c/o S. Leslie Misrock Pennie & Edmonds**
**1155 Avenue of the Americas**
**New York, NY 10036(US)**

(72) Inventor: **Hayes, Michael Edwards**
**71 Oak Lane**
**Fernandina Beach Florida 32034(US)**
Inventor: **Holzner, Günter**
**15, chemin des Palettes**
**CH-1212 Grand-Lancy(CH)**

(74) Representative: **Salvadori, Giuseppe, Dr.**
**c/o Firmenich S.A. Case Postale 239**
**CH-1211 Genève 8(CH)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

This invention relates to skin and hair care products suitable for cleansing and imparting other beneficial effects to human skin and hair. More particularly, the invention relates to soaps and shampoos containing bioemulsifiers as effective ingredients.

Biological materials have been incorporated into numerous consumer products for numerous purposes. For instance, biopolymeric materials, such as polysaccharides, proteins and nucleic acids have been added to products as diverse as paints, foods, skin creams and cleaning agents in which they serve such diverse functions as thickeners, suspending agents, moisturizers and conditioners. Certain specific biological materials have been incorporated into skin and hair care products for human use.

For example, chemically-modified glycolipids, specifically hydroxypropyl-etherified sophorolipid esters, have been used in cosmetic compositions for skin and hair care. The sophorolipids used as starting materials are the products of a yeast, Torulopsis bombicola. The chemically-modified sophorolipids improve the setting capacity of hair setting lotions and hair sprays and influence the lathering characteristics of shampoos. Hair washed with sophorolipid-containing shampoos retains a moist finishing touch. These various effects are described in US patents Nos. 4,297,340 and 4,318,901. The same chemically-modified sophorolipids can serve as moisturizing, conditioning and protective agents in skin creams and lotions [US patents Nos. 4,297,340; 4,305,961 and 4,309,447], in stick-shaped cosmetics, such as lipsticks and eyeshadows [US patent No. 4,305,929], and in pressed powder cosmetics [US patent No. 4,305,931].

A microbial biopolymer produced by Bacillus polymyxa has been proposed as a useful ingredient in certain cosmetics and shampoos [US patents Nos. 4,329,448; 4,357,423 and 4,393,089]. This biopolymer is a heteropolysaccharide comprising glucose, galactose, mannose, glucuronic acid and fucose. In the foregoing patents if is suggested that such biopolymer can be used in anti-dandruff shampoos as a suspending agent for anti-dandruff ingredients, in other shampoos and shower washes as a gelling agent, and in hand creams as an emulsion stabilizer.

A beer concentrate comprising a mixture of proteins and polysaccharides has been used as an ingredient in shampoos. The beer concentrate acts as a conditioner and also imparts body to hair shampooed with the formulations disclosed in US patent No. 3,998,761. The use of mono-, di- and polysaccharides in shampoos has been described in US patent No. 4,364,837. It is reported that the presence of saccharides in shampoos stabilizes the shampoos and also enhances foam quality. The saccharides thicken the shampoo and act as suspending agents for grooming ingredients in the shampoo. Preferred saccharides were reported to be corn and potato syrups containing glucose and di- and polysaccharides of glucose.

Many microbes can utilize hydrocarbon as their sole source of carbon for growth and energy production. The hydrocarbon substrates may be linear, branched, cyclic or aromatic. In order to rapidly assimilate such water-insoluble substrates, the microbes require a large contact area between themselves and the oil. This is achieved by emulsifying the oil in the surrounding aqueous medium. Hydrocarbon degrading microbes frequently synthesize and excrete surface active agents which promote such emulsification. Some of the microbial surface active and interfacially active agents that have been reported in the literature are listed in Table I.

## TABLE  I

### Microbial surface active compounds

| Structural type | Producing microorganism(s) |
|---|---|
| Carbohydrates-Lipids | |
| Trehalose-Lipids | Nocardia, Mycobacterium, Corynebacterium, Arthrobacter |

2

Table I  (continuation)

| Structural type | producing microorganism(s) |
|---|---|
| Rhamnose-Lipids | Pseudomonas aeruginosa |
| Sophorose-Lipids | Torulopsis spp. |
| Polysaccharide-Lipid | Candida tropicalis, Acinetobacter calcoaceticus |
| Amino Acid-Lipids | |
| Lipopeptides | Bacillus, Streptomyces, Corynebacterium, Mycobacterium |
| Ornithine-Lipids | Pseudomonas, Thiobacillus, Agrobacterium, Gluconobacter |
| Phospholipids | Thiobacillus, Corynebacterium, Candida, Micrococcus |
| Fatty Acids/Neutral Lipids | Pseudomonas, Mycococcus, Penicillium, Aspergillus, Acinetobacter, Micrococcus, Candida |

Gutnick et al. discovered that Acinetobacter calcoaceticus ATCC 31012 (deposited with the American Type Culture Collection, Rockville, MD and also known in the literature as RAG-1) produces interfacially active extracellular protein-associated lipopolysaccharide biopolymers called emulsans. The emulsans are polyanionic materials. Acinetobacter calcoaceticus ATCC 31012 produces α-emulsans when grown on ethanol or fatty acid salts [US patents Nos. 4,230,801; 4,234,689 and 4,395,354] and β-emulsans when grown on crude oil or hexadecane [US patent No. 3,941,692]. The α-emulsans and β-emulsans, which differ in their respective lipid content, can be derivatized to an O-deacylated form called psi-emulsans [US patent No. 4,380,504]. The α-emulsans, β-emulsans and psi-emulsans can be deproteinized to yield apo-α-emulsans, apo-β-emulsans and apo-psi-emulsans, respectively [US patents Nos. 4,311,830; 4,311,829 and 4,311,831, respectively]. The emulsans exhibit exceptional bioemulsifier activity especially with hydrocarbon substrates which contain both aliphatic and cyclic or aromatic components.

In addition to acting as bioemulsifiers, the emulsans are capable of interfering with the adherence of microorganisms to certain surfaces. Recently, Rosenberg et al. [Infect. Immun. 39(3): 1024-28 (1983)] have shown that emulsan can markedly inhibit the adherence of Acinetobacter calcoaceticus ATCC 31012 and Acinetobacter calcoaceticus BD 413 [Taylor and Juni, J. Bacteriol. 81: 688-93 (1961)] and Streptococcus pyogenes M-5, either to human buccal epithelial cells or to octane. The degree of interference with adherence was the same, whether the emulsan was used to prevent binding or added later, to desorb already bound bacteria.

Gutnick et al. have also reported that a phage-resistant mutant of Acinetobacter calcoaceticus ATCC 31012 produces a highly viscous, inactive (i.e., non-bioemulsifying) derivative of the emulsans which is nevertheless cross-reactive with antibodies raised against emulsan ["Emulsan Production in Acinetobacter RAG-1," in : Advances in Biotechnology, Vol. III, Proceedings of the Sixth International Fermentation Symposium, London, Canada (1980), M. Moo-Young (ed.), pp. 455-459].

It is an object of the present invention to provide skin and hair care products with desirable cleansing properties and which impart certain beneficial effects to human skin and hair. It is a further object of the invention to provide soaps with creamy feeling foams and which leave the skin feeling smooth and creamy after use. It is another object of the invention to provide soaps which soothe and/or ameliorate certain common dermatological problems such as acne, eczema, psoriasis and razor burn.

Still another object of the invention is to provide shampoos with improved degreasing abilities and with enhanced cleansing power for residues left on hair by fixative agents. It is a further object of the invention to provide shampoos which leave hair in a conditioned state after washing and which impart shine to the hair. Another object of the invention is to provide shampoos capable of improving or eliminating such common scalp and hair problems as dandruff, psoriasis/eczema of the scalp and static build-up following blow-drying or combing.

We have now discovered that the above objects can be achieved by soaps and shampoos comprising microbial bioemulsifiers, preferably lipopolysaccharides produced by certain members of the Acinetobacter genus, in standard soap and shampoo bases, wherein said bioemulsifiers are used in a defined range of concentrations.

The invention also contemplates the inclusion of emulsans and other microbial bioemulsifiers in consumer products such as body lotions, body creams, hand cleaners, ointments, wound care products and dentifrices.

Thus, it is an object of the present invention to provide a composition containing from 0.02 to 0.5% by weight of a bioemulsifier produced by a bacterium of the Acinetobacter calcoaceticus species, for its use as an active pharmaceutical substance.

The invention further provides compositions as defined above which are specifically adapted to be used as antibacterial or antimicrobial agents. According to preferred embodiments of the invention, there are provided compositions for topical use in the therapeutical treatment of dermopathological skin or scalp conditions, namely for use in the treatment of acne, eczema, dermatitis, psoriasis, dandruff or razor burn.

According to the invention, the compositions to be used as defined above are pharmaceutical compositions containing as active principle 0.02 to 0.5% by weight of a bioemulsifier produced by a bacterium of the Acinetobacter calcoaceticus species, in the form of a cream, lotion, shampoo, soap, dentifrice, ointment or wound care product, or cosmetic compositions containing from 0.02 to 0.5% by weight of a bioemulsifier produced by a bacterium of the Acinetobacter calcoaceticus species, in the form of a cream, lotion, shampoo, soap, dentifrice or ointment.

In the following description, the term "bioemulsifier" is defined as any biologically derived substance which, by virtue of any combination of characteristics including, but not limited to, high molecular weight, polymeric nature, highly specific three-dimensional structure, hydrophobic and hydrophilic moieties and sparing solubility in hydrocarbons, binds tightly to the hydrocarbon/water interface and essentially covers the surface of individual hydrocarbon droplets in hydrocarbon-in-water emulsions, effectively maintaining discrete droplets and preventing coalescence, and thereby imparting substantial stability to hydrocarbon-in-water emulsions. An example of a bioemulsifier is $\alpha$-emulsan.

The term "biosurfactant" is defined as any biologically derived substance which reduces the interfacial tension between water and a hydrocarbon and, as a result, reduces the energy requirement (mixing energy) for creation of additional interfacial area. An example of a biosurfactant is a glycolipid.

The term "emulsans", which reflects the polysaccharide structure of these compounds and the exceptional bioemulsifier activity of these materials, generically identifies those extracellular microbial protein-associated polyanionic lipoheteropolysaccharides produced by Acinetobacter calcoaceticus ATCC 31012 and its derivatives or mutants, which may be subdivided into the $\alpha$-emulsans and the $\beta$-emulsans. The $\alpha$-emulsans are the products of Acinetobacter calcoaceticus ATCC 31012 when grown on either ethanol or fatty acid salts as carbon source and the $\beta$-emulsans are the products when the organism is grown on crude oil or hexadecane, as determined under shake flask conditions.

The term "soap" is defined to include its common meaning of an alkali salt of a fatty acid, as well as detergents and so-called waterless cleaners.

The term "shampoo" is defined to include pourable water-based shampoos, gel shampoos, cream shampoos and so-called dry shampoos.

The water-soluble microbial interfacially active agents for use in the soaps and shampoos of this invention are any microbial substances which function as bioemulsifiers, i.e., substances which, by virtue of such characteristics as large molecular weight, polymeric nature, highly specific three-dimensional structure, hydrophobic and hydrophilic nature, and sparing solubility in oil, effectively cover the oil/water interface maintaining discrete, individual oil droplets in oil-in-water emulsions thereby substantially stabilizing emulsions from coalescence. Among the preferred bioemulsifiers are lipoheteropolysaccharide biopolymers produced by bacteria of the genus Acinetobacter and the genus Arthrobacter, and in particular, those produced by strains of Acinetobacter calcoaceticus. Such Acinetobacter lipoheteropolysaccharide biopolymers include polyanionic lipoheteropolysaccharide biopolymers, α-emulsans, β-emulsans, psi-emulsans, apo-α-emulsans, apo-β-emulsans and apo-psi-emulsans produced by Acinetobacter calcoaceticus ATCC 31012 and described in US patents Nos. 4,395,353; 4,395,354; 3,941,692; 4,380,504; 4,311,830; 4,311,829 and 4,311,831, respectively. Such Acinetobacter biopolymers also include the biopolymers produced by Acinetobacter calcoaceticus BD4 [Taylor and Juni, J. Bacteriol. 81 : 688-693 (1961), and Acinetobacter calcoaceticus NRRL-15616, as well as those produced by Acinetobacter calcoaceticus, strains NS-1 (NRRL B-15847), NS-4 (NRRL B-15848), NS-5 (NRRL B-15849), NS-6 (NRRL B-15860) and NS-7 (NRRL B-15850). The foregoing "NS" strains have been deposited at the Northern Regional Research Center, Peoria, IL and have been assigned the foregoing NRRL accession numbers. The "NS" strains of Acinetobacter calcoaceticus are described by Sar and Rosenberg, Current Microbiol. 9(6): 309-14 (1983). Particularly preferred Acinetobacter lipoheteropolysaccharide biopolymers are the α-emulsans, the production of which is further described in US patents Nos. 4,230,801 and 4,234,689. The α-emulsans are characterized by a Specific Emulsification Activity of about 200 units per milligram or higher, where one unit per milligram of Specific Emulsification Activity is defined as that amount of emulsifying activity per milligram of bioemulsifier which yields 100 Klett absorption units using a standard hydrocarbon mixture consisting of 0.1 ml of 1:1 (v/v) hexadecane/2-methylnaphthalene and 7.5 ml of Tris-Magnesium buffer.

In a preferred embodiment, emulsans produced by Acinetobacter calcoaceticus ATCC 31012 are included in soap and shampoo compositions in a concentration ranging from 0.04 to 0.2% by weight.

In addition to Acinetobacter products which function as bioemulsifiers, other Acinetobacter-derived materials are suitable for use in the soaps and shampoos of this invention. Certain strains of Acinetobacter calcoaceticus are capable of producing materials that exhibit little, if any, bioemulsifier activity, yet are cross-reactive with antibodies specific for Acinetobacter calcoaceticus emulsifiers. Such materials can be used as viscosity agents and are called viscoemulsans.

The term "viscoemulsan" is defined as any lipoheteropolysaccharide, derived from a bacterium of the genus Acinetobacter and immunologically cross-reactive with antibodies against an Acinetobacter-derived bioemulsifier, which does not exhibit significant bioemulsifier activity, yet, by virtue of any combination of characteristics, including but not limited to, high molecular weight, polymeric nature and highly specific three-dimensional structure, exhibits the ability to increase the viscosity or otherwise alter the rheology of solutions or dispersions in which it is dissolved or suspended, respectively. An example of a viscoemulsan is the emulsan-cross-reactive material produced by Acinetobacter calcoaceticus ATCC 31926 which is cross-reactive with antibodies against emulsans produced by Acinetobacter calcoaceticus ATCC 31012. In solution, this viscoemulsan causes an increase in viscosity and thus is potentially useful in such products as liquid soaps and shampoos.

The ability of the emulsans, particularly α-emulsan, to stabilize emulsions makes them ideal ingredients in cleansing agents for the removal of greasy dirt and oils, including sebum, from human skin and hair. The principal factors controlling emulsion stability are electrostatic (charge) effects and steric effects. The properties of emulsans lend themselves to optimal exploitation of these mechanisms. Their large molecular weight and highly specific three-dimensional structure result in an efficient coverage of the oil/water interface. Hence, the oil droplets in oil-in-water emulsions are essentially "coated" with emulsan molecules. This effectively prevents oil-to-oil contact when collisions occur between adjacent droplets. Simultaneously, the polyanionic nature of emulsans cause the surfaces of emulsion droplets to be negatively charged which creates repulsive forces and significantly decreases the collision frequency between hydrocarbon droplets. In addition, the absence of multimolecular emulsan micelles in the water phase and the lack of emulsan solubility in the hydrocarbon phase provides an efficient migration and attachment of the emulsan molecules to the oil/water interface. The overall result is that coalescence of the oil droplets is very significantly retarded. This in turn means that any grease or oils to be removed from the skin or hair will remain in an emulsified state and can be easily rinsed off with water.

Soap compositions to which bioemulsifiers can be added advantageously are standard toilet soap bases, normal or superfatted, comprising alkali metal salts of fatty acids from such sources as, for example, tallow, lard, coconut oil, palm oil and/or other edible oils. Other ingredients may also be added, for example, perfumes or medications. The bioemulsifier may be added to the soap bases in relatively low concentrations and still achieve desirable results. For technical grade α-emulsan, the product of Acinetobacter calcoaceticus ATCC 31012, a concentration range of 0.02% to 0.5% by weight is preferred. Higher

5

quantities of technical grade α-emulsan may leave skin feeling dry after washing.

Shampoo compositions to which bioemulsifiers can be added advantageously are standard shampoo bases which comprise synthetic detergents. A store-bought baby shampoo has served as a suitable shampoo composition. Exemplary of ingredients that may be found in standard shampoo bases in a variety of combinations are fatty acid polyglycol esters (e.g., glycol distearate), fatty acid diethanolamides (e.g., cocamide DEA), neutralized salts of alkyl ether sulfates (e.g., sodium lauryl ether sulfate), cocoamphocarboxyglycinate, sugars (e.g., fatty acid sarcosides such as sodium lauroyl sarcosinate), salts (e.g., sodium chloride), buffering agents and preservatives (e.g., citric acid), biocides (e.g., methylchloro- and methylisothiazolinone), fatty acid/amine condensation products (e.g., polyglycol-polyamine condensation products), salts of polypeptides (e.g., salts of hydrolyzed animal protein) and water. Other ingredients such as perfumes and grooming agents can also be present. The bioemulsifier may be added to the shampoo bases in small amounts. For technical grade α-emulsans, a concentration range of 0.02% to 0.5% by weight can be used. A preferred concentration range is between 0.05% to 0.15% by weight. Depending on the grade of bioemulsifier used, it may be desirable to add other ingredients to the shampoo. For example, technical grade α-emulsan, though dispersible, is not completely soluble in all shampoo raw materials. Therefore, it is desirable to use a pearlescing agent to make an opaque, pearlescent shampoo. It should be appreciated that grades of bioemulsifiers can be produced that are freely soluble in shampoo raw materials and can be used in translucent shampoos.

The soaps and shampoos of the present invention can be used to impart desirable characteristics to human skin and hair. Washing with soaps containing bioemulsifiers, particularly α-emulsan, can make skin smooth and creamy to the touch. Shampooing with shampoos containing bioemulsifiers can make hair conditioned, shiny and free of static build-up. Because of its superior cleansing and degreasing properties, bioemulsifier-containing shampoo can increase the time interval between washings. Hair fixatives such as sprays, laquers, creams, etc. can be removed easily with bioemulsifier-containing shampoos.

In addition to imparting aesthetically pleasing characteristics to skin and hair, the bioemulsifier-containing soaps and shampoos of the present invention, when used regularly, can bring about certain hygienically and medically beneficial effects. Soaps and shampoos containing emulsans have demonstrably beneficial effects on such common skin and scalp conditions as dermatitis, dandruff, psoriasis, eczema, acne and razor burn and are therefore potentially useful as medicaments for the treatment and/or control of these and other dermatological conditions. The mechanism by which these beneficial effects are brought about is as yet unknown. For a condition like acne, which is often associated with overproduction of sebum by sebaceous glands and the proliferation of microorganisms that feed on sebum, e.g., Corynebacterium acnes (which in turn lead to the blocked ducts, papules, pustules and general inflammation that are characteristic of the condition), it may be speculated that the emulsans effectuate improved removal of sebum from the skin, thereby preventing the bacterial growth that is symptomatic, if not causative, of the disease. Alternatively, since it has been demonstrated in some instances that emulsans have the ability to desorb bacteria from epithelia, it may also be speculated that the soaps and shampoos have their beneficial effects as a result of their desorbing disease-causing or disease-related microorganisms from the skin. The desorptive effect has lead to the contemplation of the use of emulsans in wound treatment compositions and other products (e.g., wound dressings) to prevent or inhibit microbial organisms from infecting wounds.

Other mechanisms besides degreasing and desorbing of bacteria may explain the beneficial effects of bioemulsifier-containing soaps and shampoos. The proposal of these two mechanisms is by no means intended as a limitation on the properties a bioemulsifier must have to be advantageously included in a soap or shampoo.

## PREPARATION OF TECHNICAL GRADE EMULSAN

The α-emulsans produced by Acinetobacter calcoaceticus ATCC 31012 during fermentation on ethanol are known bioemulsifiers as described in US patent No.4,395,354, supra. The α-emulsans used in the soap and shampoo compositions described infra were technical grade materials, prepared in either of two ways. Both methods of preparation involved enzyme treatment and drying but differed in the order in which these steps were performed.

By one method, centrifuged (approximately 90% cell-free) fermentation broth containing α-emulsans resulting from a fermentation of Acinetobacter calcoaceticus ATCC 31012 in ethanol medium was drum-dried and the resulting material was treated in the following manner prior to use. A 10% by weight suspension of the material, so-called technical grade α-emulsan, was prepared in deionized water and heated to 50-60° C while continuously stirring. The pH of the suspension was adjusted to pH 8.5 by adding 50% by weight sodium hydroxide (diluted, if necessary). Protease enzyme (NOVO Industries, 1.5 M Alcalase) was added at a level of 1 part protease: 500 parts solid α-emulsan. The mixture was allowed to remain at 50-60° C while being stirred for about three hours. Reactions were run to completion as judged by the absence of visible precipitable α-emulsans following centrifugation of the reaction mixture. After completion of the enzyme treatment, the reaction mixtures were raised to approximately 70° C to denature the protease and stop its activity. The solutions were cooled to room temperature and Cosan PMA-30 (Cosan Corporation), a preservative, was added at a level of 1 part Cosan: 500 parts α-emulsan solution.

By another method, enzyme treatment of the α-emulsans was performed prior to drum drying according to the following protocol. Fermentation broth containing α-emulsans resulting from a fermentation of Acinetobacter calcoaceticus ATCC 31012 in ethanol medium was centrifuged to remove approximately 90% of the bacterial cells. To the centrifuged broth, protease enzyme (as previously described) was added in a ratio of 1 gram protease: 500 units per milligram of Specific Emulsification Activity (where one unit per milligram of Specific Emulsification Activity is defined as that amount of emulsifying activity per milligram of bioemulsifier which yields 100 Klett absorption units using a standard hydrocarbon mixture consisting of 0.1 ml of 1.1 (v/v) hexadecane/2-methylnaphthalene and 7.5 ml of Tris-Magnesium buffer). The protease reaction was run to completion as described supra. The protease-treated centrifuged broth was then evaporated to a 10% (w/v) slurry of α-emulsans. The slurry was sprayed dried and the resulting material is also referred to as technical grade α-emulsan.

Bar soaps were formulated with α-emulsans prepared by each of the foregoing procedures. The method of preparation of α-emulsans made no apparent difference in the final bar soap product, as evaluated in the laboratory on the basis of a qualitative criterion, feel to the hands. The α-emulsans prepared by the second procedure described supra were used in the soaps and shampoos distributed to individuals to be evaluated as personal skin and hair care products and as beneficial agents for such skin conditions as acne, psoriasis, eczema, dandruff, etc.

The α-emulsans prepared by the methods described above were not added directly to the soap bases described hereinafter although they can be. Instead, the α-emulsans were first formulated into a composition comprising non-ionic surfactants and water. The formula for the composition was the following: 30% Triton X-114 (an ethoxylated alkyphenol commercially available from Rohm & Haas Co., Philadelphia, PA); 10% Tergitol TMN-6 (an ethoxylated alcohol commercially available from Union Carbide Corp., Danbury, CT); 3% α-emulsan and 57% water (percentages are on a weight basis). Also, this formulation was added to the shampoo bases described in the following examples. Shampoos containing the formulation were used in the shampoo testing reported in example 3b) below. However, the subjects of examples 4, 5 and 6 presented hereinafter used shampoos to which α-emulsan had been added directly.

Trade marks are acknowledged as such.

## Example 1

### Emulsan in toilet soap

#### a) Bar soap composition

Standard toilet soap base (Steinfels, Switzerland) was used which contained, approximately, 10% coconut oil; 40% beef tallow and 50% pork grease. A superfatted soap base was also used. The emulsan formulation described above was readily incorporated together with color and perfume (Firmenich SA, Switzerland) by intensive mixing on a three-roller mill and final extrusion in a laboratory soap plodder. The final concentration of α-emulsan in the bar soap ranged from 0.05% to 0.3% on a weight basis. At these low concentrations, there was no detectable odor of α-emulsan. The use of concentrations of α-emulsan higher than 0.3% resulted in soap bars that left a dry feeling to the skin after washing. This drying effect is probably due to the presence of the synthetic detergents in the emulsan formulation added to the soap bases.

#### b) Bar soap testing

A group of ten panelists were given soap bars with and without α-emulsan as an ingredient. The concentration of α-emulsan in soap bars containing the bioemulsifier was 0.15% by weight. Each panelist alternated soap bars on a daily basis, one day using the soap bar containing α-emulsan and the next day using the control soap bar without α-emulsan. The panelists were asked to evaluate and compare the soap bars based on several qualitative, subjective criteria, including foam formation on hands, foam consistency, feeling on wet skin and feeling on dry skin. No special soil was applied to the panelists' hands. The panelists reported that there was no significant difference in foaming properties of the soap bars, although the $\alpha$-emulsan-containing soap bars lathered into a perceptibly creamier feeling foam. Also, seven out of ten panelists indicated their skin had a better, creamier feeling after washing with the $\alpha$-emulsan-containing soap bars.

Example 2

Effect of emulsan-containing soap bars on skin conditions

a) Effect of emulsan-containing soap bars on acne

Several individuals with acne and acne-like conditions washed the affected parts of their bodies with $\alpha$-emulsan-containing bar soap for varying periods of time and experienced certain beneficial effects as the result of such use. Individual cases are presented as follows:

One subject was a teenage female who had a moderately severe acne-like condition on her back. The condition had not been medically diagnosed and is therefore referred to as an "acne-like" condition. The condition had been present for about one year during which time the subject had used various cleansers on her back. The use of $\alpha$-emulsan-containing bar soap (0.15% w/v technical grade $\alpha$-emulsan) on a daily basis during bathing coincided with the prompt, gradual disappearance of the subject's acne-like condition. In less than one week, the acne-like condition was gone. When the supply of $\alpha$-emulsan-containing soap was exhausted, the condition returned. The subject was given another supply of $\alpha$-emulsan-containing bar soap (0.05% w/v technical grade $\alpha$-emulsan) and again the acne-like condition went away upon daily use of the soap. The subject also noted that the soap left her skin feeling smooth.

Another subject was a teenage male with a medically-diagnosed severe case of acne on his face and back. The acne had been present for about one to two years during which time the subject had used various cleansers on his face and back. The use of $\alpha$-emulsan-containing bar soap (0.15% w/v technical grade $\alpha$-emulsan) on a daily basis during bathing coincided with a prompt and gradual improvement of the acne on the subject's face and back. In approximately one week, the condition had changed from a severe to slight case of acne; this improvement was noted by a physician.

Another subject was a teenage female who had a moderately severe acne-like condition (not medically diagnosed) on her face. The condition had been present for several months during which time the subject had used various cleansers on her face. The use of $\alpha$-emulsan-containing bar soap (0.05% w/v technical grade $\alpha$-emulsan) on a daily basis during bathing coincided with a noticeable improvement in the acne-like condition. In less than one week, the acne-like condition changed from moderate to very slight. The subject further noted that the soap left her skin feeling smooth.

Another subject was a male in his early twenties with a moderately severe acne-like condition (not medically diagnosed) on his face and chest. The condition had been present for several years. Upon daily washings of the affected body parts with $\alpha$-emulsan-containing bar soap (0.18% w/v technical grade $\alpha$-emulsan), the subject experienced the following results. The acne-like condition on the chest improved significantly within 3 days and cleared up completely within two weeks of use. With regard to the acne-like condition on the face, the subject observed a flare-up of the condition within the first week of use. A stinging sensation upon washing was also experienced. Thereafter, upon continued use of the soap, the acne-like condition on the subject's face completely disappeared within 7 days.

b) Effect of emulsan-containing soap-bars on razor burn

The subjects were two adult males with intermittent yet severe cases of razor burn on their necks. Use of $\alpha$-emulsan-containing bar soap (0.05% w/v technical grade $\alpha$-emulsan) for showering immediately prior to shaving significantly reduced the incidence of razor burn. Results were immediately observed after use of the bar soap. Any razor burn experienced was very slight.

Example 3

Emulsan in shampoo

a) Shampoo base compositions

Emulsan shampoos were formulated by adding 0.1-0.15% $\alpha$-emulsan to two shampoo base compositions developed for use with the technical grade α-emulsan described in the introduction. Formulas for the shampoo bases, designated DAM 82/25 and DAM 83/12, are presented in Tables II and III, respectively. Trade names and suppliers of commercially available products are also provided.

## TABLE II

### Composition of shampoo base DAM 82/25

| Ingredient | Per cent | Commercial Product and Supplier |
|---|---|---|
| Glycol distearate | 0.5 | Genapol PMS, Hoechst Laboratories (Puteaux, France) |

Table II (continuation)

| Ingredient | Per cent | Commercial Product and Supplier |
|---|---|---|
| Cocamide Diethanolamide | 2.5 | Comperlan KD, Henkel Corp. (Dusseldorf, West Germany) |
| Sodium laureth sulfate | 21.0 | Texapon N 25, Henkel Corp. |
| Demineralized water | 44.3 | |
| Methylchloroisothiazolinone and methylisothiazolinone | 0.1 | Kathon CG, Rohm & Haas Co. (Philadelphia, PA) |
| Citric acid | 0.1 | |
| Sodium chloride | 0.5 | |
| Sodium lauroyl sarcosinate | 5.0 | Medialan LD, Hoechst Laboratories |
| Polyethylene glycol-15 tallow polyamine | 1.0 | Polyquart H, Henkel Corp. |
| Hydrolyzed animal protein | 4.0 | Nutrilan L Liquid, Grunau (Bavaria, West Germany) |
| Cocoamphocarboxy-glycinate/ Sodium laureth sulfate/ Sodium lauryl sulfate | 20.0 | Amphotensid 9M, Zschimmer & Schwarz GmbH & Co. (Lahnstein, West Germany) |
| Perfume | 1.0 | Firmenich SA (Geneva, Switzerland) |

TABLE III

| Composition of shampoo base DAM 83/12 | | |
|---|---|---|
| Ingredient | Per cent | Commercial Product and Supplier |
| Citric acid | 0.2 | |
| Methylchloroisothiazolinone and methylisothiazolinone | 0.1 | Kathon CG, Rohm & Haas Co. (Philadelphia, PA) |
| Water | 45.7 | |
| Sorbitol 70% | 5.0 | |
| Polyethylene glycol alkyl ether 60% | 15.0 | Triton CB 110, Rohm & Haas Co. |
| Cocoamphocarboxy-glycinate/Sodium laureth sulfate/Sodium lauryl sulfate | 30.0 | Amphotensid 9M, Zschimmer & Schwarz GmbH & Co. (Lahnstein, West Germany) |
| Cocamide Diethanolamide | 3.0 | Comperlan KD, Henkel Corp. (Dusseldorf, West Germany) |
| Pearly agent | 0.5 | Product GM 4055, Zschimmer & Schwarz GmbH & Co. |
| Perfume | 0.5 | Firmenich SA (Geneva, Switzerland) |

DAM 82/25 was prepared in the following manner.

The glycol distearate, cocamide diethanolamide and sodium laureth sulfate were heated to approximately 65-70° C and mixed until homogeneous; this mixture is referred to as Part A. The remaining ingredients, except for the perfume, were combined to make a mixture referred to as Part B. Slowly, a little at a time, Part B was added into Part A, while mixing, until the combined Parts A and B were perfectly homogeneous. Finally, the perfume was added. DAM 83/12 was prepared by mixing all the ingredients listed in Table III, one after the other, according to the sequence of ingredients listed.

b) Shampoo testing

Shampoo (DAM 82/25) containing technical grade α-emulsan at concentrations in the range of approximately 0.1-0.15% yielded the best results. Higher concentrations of this material made hair dull and unpleasant to touch.

The α-emulsan shampoo was used to wash hair containing large amounts of residues from fixative agents such as laquers, creams, hair sprays, etc. Hair containing such residues is normally difficult to wash; other shampoos leave fine white residues that adhere to combs when the hair is combed after washing. It was found that the addition of 0.15% technical grade α-emulsan to the composition designated DAM/25, described in a) resulted in a shampoo with better cleaning power for the previously mentioned residues. Furthermore, no white residues were observed on combs after combing hair that had been washed with the α-emulsan shampoo.

Hair washed with the α-emulsan shampoo was evaluated with regard to regreasing, i.e, the length of the time interval before washing was again required. For some test persons the interval between washings was 30% longer than normal; for others, the interval between washings was twice as long as normal.

The α-emulsan shampoo was further evaluated on hair of varying thicknesses. It was observed that the shampoo imparted a slight fixative effect to fine hair. On the other hand, it was observed that the shampoo had a tendency to leave thick hair harsh and unpleasant to touch at α-emulsan concentrations higher than about 0.2%.

Example 4

Effect of emulsan-containing shampoo on scalp conditions

a) Effect of emulsan-containing shampoo on dandruff

The subject was an adult male with a moderately severe case of dandruff that had persisted for several years. The subject had never consulted a physician about his dandruff condition but had been using various commercially available medicated shampoos on his hair and scalp. In approximately one week during which the subject used DAM 82/25 shampoo containing 0.15% technical grade α-emulsan, an improvement in the condition was observed. Upon further use the dandruff had disappeared. When the supply of shampoo was exhausted, the dandruff condition returned to its former level. Resumption of use of the α-emulsan shampoo again resulted in an improvement in the condition. The subject also observed that his hair was better conditioned after having used the α-emulsan shampoo.

b) Effect of emulsan-containing shampoo on eczema/psoriasis of the scalp

The subject was an adult female with a moderate form of an eczema/psoriasis-like condition on her scalp. The condition had been medically diagnosed. This skin condition, which had been present for several years and which had been kept marginally controlled by treatments, e.g., medicated shampoos, prescribed by dermatologists, was observed to be significantly improved upon use of the α-emulsan shampoo (DAM 82/25). Results were observed in approximately one week with shampoo containing 0.18% technical grade α-emulsan. When the supply of shampoo was exhausted, the skin condition returned to its former state. Unbeknownst to her, the subject was next given a placebo shampoo which did not contain any α-emulsan. Within a few days, the subject, of her own initiative, reported that she was no longer observing the same beneficial effects as she had with the initial supply. The subject was then provided with a standard store-bought brand of baby shampoo to which 0.175% technical grade α-emulsan was added. Use of this shampoo resulted in significant improvement of the condition. This subject also observed that emulsan-containing shampoos left her hair feeling better conditioned.

Example 5

Conditioning effects of emulsan-containing shampoo

a) Decreased static build-up

The subject was an adult male who experienced static build-up in his hair when blow drying it after shampooing with conventional shampoos. The first time the subject used α-emulsan shampoo (DAM 82/25 containing 0.15% technical grade α-emulsan), he observed a very noticeable loss of static in his hair after blow drying. Static returned when shampoos without α-emulsan were used. Resumption of use of α-emulsan shampoo again eliminated the static build-up.

b) Improved shine

The subject was an adult female who normally experienced dullness of her hair after shampooing with conventional shampoos. However, a very noticeable improvement in "shine" was observed each time α-emulsan shampoo (0.15% technical grade α-emulsan in DAM 82/25) was used. The α-emulsan shampoo was used over a period of approximately nine months during which time the improved "shine" was continually observed.

Example 6

Effect of emulsan-containing shampoo on mildew

During a period of use of emulsan-containing shampoo (0.15% technical grade α-emulsan in DAM 82/25), mildew did not accumulate on the shower curtain. When the shampoo supply was exhausted, the mildew began to reappear on the shower curtain. Resumption of use of emulsan shampoo upon obtaining a new supply gave a gradual decrease in mildew severity.

**Claims**

1. A composition containing from 0.02 to 0.5% by weight of a bioemulsifier produced by a bacterium of the Acinetobacter calcoaceticus species, for its use as an active pharmaceutical substance.

2. A composition according to claim 1, for its use as an antibacterial or antimicrobial agent.

3. A composition according to claim 1, for its topical use in the therapeutic treatment of dermopathological skin or scalp conditions.

12

**4.** A composition according to claim 3, for its use in the treatment of acne, eczema, dermatitis, psoriasis, dandruff or razor burn.

**5.** A pharmaceutical composition containing as active principle 0.02 to 0.5% by weight of a bioemulsifier produced by a bacterium of the Acinetobacter calcoaceticus species, in the form of a cream, lotion, shampoo, soap, dentifrice, ointment or wound care product.

**6.** A cosmetic composition containing from 0.02 to 0.5% by weight of a bioemulsifier produced by a bacterium of the Acinetobacter calcoaceticus species, in the form of a cream, lotion, shampoo, soap, dentifrice or ointment.

**7.** A composition according to claim 1, 5 or 6, characterized in that the Acinetobacter calcoaceticus species is selected from the group consisting of Acinetobacter calcoaceticus ATCC 31012, Acinetobacter calcoaceticus NRRL B-15616, Acinetobacter calcoaceticus NRRL B-15847, Acinetobacter calcoaceticus NRRL B-15848, Acinetobacter calcoaceticus NRRL B-15849, Acinetobacter calcoaceticus NRRL B-15850, and Acinetobacter calcoaceticus NRRL B-15860.

**8.** A composition according to claim 1, 5 or 6, characterized in that the bioemulsifier is a lipoheteropolysaccharide selected from the group consisting of α-emulsan, apo-α-emulsan, ψ-emulsan, apo-ψ-emulsan, and β-emulsan.

**9.** A composition according to claim 6, 7 or 8, characterized in that it further contains viscoemulsan, a lipoheteropolysaccharide produced by Acinetobacter calcoaceticus ATCC 31926.

**Revendications**

**1.** Une composition contenant de 0,02 à 0,5% en poids d'un bioémulsificateur produit par une bactérie de l'espèce Acinetobacter calcoaceticus, pour son utilisation en tant que substance pharmaceutique active.

**2.** Une composition selon la revendication 1, pour son utilisation en tant qu'agent antibactérien ou antimicrobien.

**3.** Une composition selon la revendication 1, pour son utilisation topique dans le traitement thérapeutique des conditions pathologiques de la peau ou du cuir chevelu.

**4.** Une composition selon la revendication 3, pour son utilisation dans le traitement de l'acné, de l'eczéma, de la dermatite, du psoriasis, des pellicules ou du feu du rasoir.

**5.** Une composition pharmaceutique contenant comme principe actif 0,02 à 0,5% en poids d'un bioémulsificateur produit par une bactérie de l'espèce Acinetobacter calcoaceticus, sous forme d'une crème, d'une lotion, d'un shampoing, d'un savon, d'un dentifrice, d'un onguent ou d'un produit pour les soins des blessures.

**6.** Une composition cosmétique contenant de 0,02 à 0,5% en poids d'un bioémulsificateur produit par une bactérie de l'espèce Acinetobacter calcoaceticus, sous forme d'une crème, d'une lotion, d'un shampoing, d'un savon, d'un dentifrice ou d'un onguent.

**7.** Une composition selon la revendication 1, 5 ou 6, caractérisée en ce que l'espèce Acinetobacter calcoaceticus est choisie dans le groupe constitué par l'Acinetobacter calcoaceticus ATCC 31012, l'Acinetobacter calcoaceticus NRRL B-15616, l'Acinetobacter calcoaceticus NRRL B-15847, l'Acinetobacter calcoaceticus NRRL B-15848, l'Acinetobacter calcoaceticus NRRL B-15849, l'Acinetobacter calcoaceticus NRRL B-15850 et l'Acinetobacter calcoaceticus NRRL B-15860.

**8.** Une composition selon la revendication 1, 5 ou 6, caractérisée en ce que le bioémulsificateur est un lipohétéropolysaccharide choisi dans le groupe constitué par α-émulsan, apo-α-émulsan, ψ-émulsan, apo-ψ-émulsan et β-émulsan.

**9.** Une composition selon la revendication 6, 7 ou 8, caractérisée en ce qu'elle contient, en plus,

EP 0 178 443 B1

viscoémulsan, un lipohétéropolysaccharide produit par l'Acinetobacter calcoaceticus ATCC 31926.

**Patentansprüche**

1. Eine Komposition, die 0,02 bis 0,5 Gewichtsprozente eines von einer Bakterie auf der Acinetobacter calcoaceticus Art produziertem Bioemulgators enthält, zur Verwendung als aktive pharmazeutische Substanz.

2. Eine Komposition gemäß Anspruch 1, zur Verwendung als antibakterielles oder antimikrobisches Mittel.

3. Eine Komposition gemäß Anspruch 1, zur lokalen Verwendung in der therapeutischen Behandlung von dermopatologischen Haut- oder Skalpkrankheiten.

4. Eine Komposition gemäß Anspruch 3, zur Verwendung in der Behandlung von Aktie, Ekzemen, Hautentzündungen, Schuppenflechten, Kopfschuppen oder gegen die durch die Rasur gereizte Haut.

5. Eine pharmazeutische Komposition, die als aktives Prinzip 0,02 bis 0,5 Gewichtsprozente eines von einer Bakterie auf der Acinetobacter calcoaceticus Art produziertem Bioemulgators enthält, in der Form einer Creme, einer Lotion, eines Haarwaschmittels, einer Seife, einer Zahnpaste, einer Salbe oder eines Wundebehandlungproduktes.

6. Eine kosmetische Komposition, die 0,02 bis 0,5 Gewichtsprozente eines von einer Bakterie auf der Acinetobacter calcoaceticus produziertem Bioemulgators enthält, in der Form einer Creme, einer Lotion, eines Haarwaschmittels einer Seife, einer Zahnpaste oder einer Salbe.

7. Eine Komposition gemäß Anspruch 1, 5 oder 6, dadurch gekennzeichnet, daß die Acinetobacter calcoaceticus Art unter der Gruppe, die aus Acinetobacter calcoaceticus ATCC 31012, Acinetobacter calcoaceticus NRRL B-15616, Acinetobacter calcoaceticus NRRL B-15847, Acinetobacter calcoaceticus NRRL B-15848, Acinetobacter calcoaceticus NRRL B-15849, Acinetobacter calcoaceticus NRRL B-15850 und Acinetobacter calcoaceticus NRRL B-15860 besteht, ausgewählt wird.

8. Eine Komposition gemäß Anspruch 1, 5 oder 6, dadurch gekennzeichnet, daß der Bioemulgator ein Lipoheteropolysaccharid ist, welcher unter der Gruppe, die aus $\alpha$-Emulsan, apo-$\alpha$-Emulsan, $\psi$-Emulsan, apo-$\psi$-Emulsan und $\beta$-Emulsan besteht, augewählt wird.

9. Eine Komposition gemäß Anspruch 6, 7 oder 8, dadurch gekennzeichnet, daß sie zusätlich Viscoemulsan, ein von Acinetobacter calcoaceticus ATCC 31926 produziertes Lipoheteropolysaccharid, enthählt.

14